(19) Europäisches Patentamt
European Patent Office
Office européen des brevets

(11) EP 2 765 419 A1

(12) EUROPEAN PATENT APPLICATION
published in accordance with Art. 153(4) EPC

(43) Date of publication:
**13.08.2014 Bulletin 2014/33**

(21) Application number: **12838930.1**

(22) Date of filing: **05.10.2012**

(51) Int Cl.:
***G01N 33/483*** *(2006.01)* ***G01N 27/62*** *(2006.01)*
***G01N 33/66*** *(2006.01)*

(86) International application number:
**PCT/JP2012/076017**

(87) International publication number:
**WO 2013/051710 (11.04.2013 Gazette 2013/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2011 JP 2011222353**

(71) Applicants:
- **Kagoshima University**
  **Kagoshima-shi, Kagoshima 890-8580 (JP)**
- **Sumitomo Bakelite Company Limited**
  **Shinagawa-ku**
  **Tokyo 140-0002 (JP)**

(72) Inventors:
- **NATSUGOE, Shoji**
  **Kagoshima-shi**
  **Kagoshima 890-8580 (JP)**
- **UCHIKADO, Yasuto**
  **Kagoshima-shi**
  **Kagoshima 890-8580 (JP)**
- **HASHIGUCHI, Teruto**
  **Kagoshima-shi**
  **Kagoshima 890-8580 (JP)**
- **SHINCHI, Hiroyuki**
  **Kagoshima-shi**
  **Kagoshima 890-8580 (JP)**
- **MAEMURA, Kosei**
  **Kagoshima-shi**
  **Kagoshima 890-8580 (JP)**
- **MATAKI, Yuko**
  **Kagoshima-shi**
  **Kagoshima 890-8580 (JP)**
- **MORIWAKI, Norichika**
  **Tokyo 108-8559 (JP)**
- **SEKIJIMA, Masaru**
  **Tokyo 108-8559 (JP)**
- **SHIMAOKA, Hideyuki**
  **Tokyo 140-0002 (JP)**
- **SAKAGUCHI, Midori**
  **Tokyo 140-0002 (JP)**
- **FUKUSHIMA, Masao**
  **Tokyo 140-0002 (JP)**
- **IGARASHI, Kota**
  **Tokyo 140-0002 (JP)**
- **ABE, Hiroki**
  **Tokyo 140-0002 (JP)**
- **AIHARA, Taichi**
  **Tokyo 140-0002 (JP)**

(74) Representative: **Müller-Boré & Partner**
**Patentanwälte PartG mbB**
**Friedenheimer Brücke 21**
**80639 München (DE)**

# (54) DIAGNOSTIC MARKER FOR GASTROINTESTINAL CANCER, AND TESTING METHOD FOR GASTROINTESTINAL CANCER

(57) A diagnostic marker for digestive organ cancer according to the present invention is used for determining whether or not to have a digestive organ cancer. The diagnostic marker for digestive organ cancer contains at least one of N-binding type sugar chains released from a glycoprotein contained in blood and represented by the following formulas (1) to (6). This makes it possible to provide a diagnostic marker for digestive organ cancer capable of being used for an inspection method of easily determining whether or not to have a digestive organ cancer at an early stage, and an inspection method for digestive organ cancer of easily determining whether or not to have a digestive organ cancer at an early stage.

EP 2 765 419 A1

(1) (2) (3) (4) (5) (6)

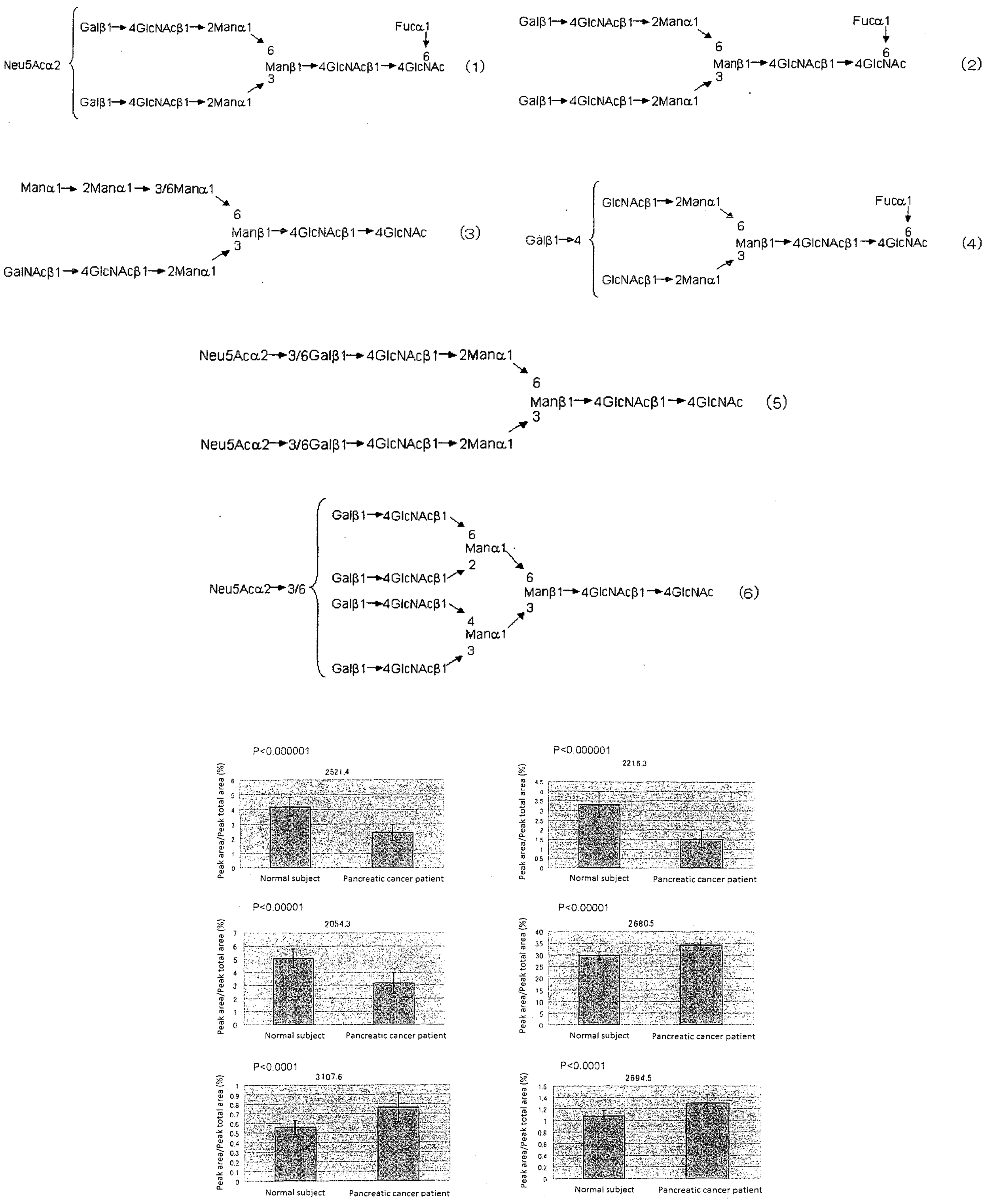
P<0.000001
2521.4
P<0.000001
2216.3
P<0.00001
2054.3
P<0.00001
26805
P<0.0001
3107.6
P<0.0001
2694.5
Peak area/Peak total area (%)
Normal subject
Pancreatic cancer patient

FIG. 1

## Description

### TECHNICAL FIELD

The present invention relates to a diagnostic marker for digestive organ cancer and an inspection method for digestive organ cancer.

### RELATED ART

Digestive organs are organs having functions of receiving, conveying and digesting foods, and functions of absorbing and excreting nutrients. Among the digestive organs, pancreas is an organ having a length of about 15 cm and located at the back side of stomach. Major diseases of the pancreas are pancreatic cancer and pancreatitis. The pancreatic cancer is particularly known as one of cancers with increased mortality rate among Japanese in recent years.

Causes of the pancreatic cancer have not been completely identified. Risk factors of the pancreatic cancer are considered to be excessive consumption of animal fat or animal protein due to Westernized dietary habits, excessive consumption of alcohol, smoking and the like. In addition, people each having a history of chronic pancreatitis, pancreatolithiasis, diabetes or acute pancreatitis are also considered to belong in a high-risk group.

The pancreatic cancer is a type of cancer which is initiated in cells each having an exocrine function, particularly, in cells of a pancreatic duct through which a pancreatic juice flows. 90% or more of the pancreatic cancer is classified into this type of cancer. Since the pancreatic cancer is highly malignant and metastasizes to other organs at an early stage, early detection of the pancreatic cancer becomes one of important issues.

However, the pancreas is surrounded by many organs such as stomach, duodenum, spleen, small intestine, large intestine, liver and gallbladder, and is at retroperitoneum. For this reason, it is very difficult to detect the cancer at an early stage even by using various kinds of diagnostic imaging. Therefore, now the pancreatic cancer is often discovered as an advanced cancer.

Further, areas where stomach cancer occurs with high frequency are Japan and South East Asia. Mortality caused by such a cancer in these areas is second in the world. Prognosis of the stomach cancer is improved due to progress in diagnostic techniques and cures. However, prognosis of advanced stomach cancer is far from good. Especially, a five year survival rate of prognosis of stomach cancer which permeates gastric-serosa is low at 35%.

Peritoneal dissemination is one of major causes of recurrence which occurs after curative resection of the advanced cancer. A curative effect on the recurrence of the peritoneal dissemination is also being achieved due to improvement of chemotherapy. However, a five year survival rate of a patient who has experienced recurrence of the peritoneal dissemination is still low. It is well known that a lot of steps and a lot of genes are involving in a mechanism of the peritoneal dissemination of the stomach cancer. There are reports that an adhesion molecule-related gene, an apoptosis-related gene and the other gene are deeply involving in the peritoneal dissemination of the stomach cancer. Further researches are also necessary on a mechanism of metastasis of the stomach cancer including the peritoneal dissemination of the stomach cancer.

Moreover, about half of the esophageal cancer occurs at middle intra-thoracic esophagus. In Japan, 90% or more of the esophageal cancer is squamous cell carcinoma. Morbidity and mortality of the esophageal cancer of males are higher than that of females, and are five times or more higher than that of females. As for occurrence of the esophageal cancer, involvement of drinking and smoking is reported before. Further, in recent years, as for the occurrence of the esophageal cancer, involvement of ALDH2 is also reported. The esophageal cancer is a bad-prognosis disease with high invasion in a surgical operation thereof.

In recent years, cases discovered as an early stage cancer are increasing, and thus cases capable of being completely cured by an endoscopic treatment are also increasing. An effect of a chemical radiotherapy to the early stage cancer is also achieved, and treatment results are improved. However, prognosis of an unresectable advanced cancer is still bad. Therefore, there is a demand for establishing a new screening system of the early stage cancer and improving a chemotherapy in the future.

In this regard, as a method of inspecting and diagnosing the cancer which has been developed so far, there is a method of measuring a tumor marker.

Examples of a serum tumor marker for diagnosing the pancreatic cancer include CA19-9 (see Non-patent document 1), Dupan-2 (see Non-patent document 2), CA-50 (see Non-patent document 3), Span-1 (see Non-patent document 4) and the like. Further, some patent publications disclose methods of inspecting and diagnosing the pancreatic cancer by using genes specifically expressed in tumor cells as a marker.

To date, PANCIA and PANCIB (see Patent document 1) and KCCR13L (see Patent document 2) have been disclosed as pancreatic cancer marker genes. Further, since pancreatic cancer cells have a DNA amplification or deletion of a specific chromosomal site, a method of diagnosing the pancreatic cancer by detecting the above specific amplification or deletion is also proposed.

Further, as for the stomach cancer, there is a method of using a variation of expression of known carboxyterminal non-triple strand telopeptide of type I collagen (ICTP) as a marker. Further, an appropriate diagnostic marker of the advanced stomach cancer, especially, scirrhous stomach cancer is proposed (see Patent document 3). A method, in which the number of a demethylated DNA existing in a DNA repeating sequence obtained from a cancer part or non-cancer part organization sample, and then it is determined whether or not prognosis of various cancer diseases including the stomach cancer is good based on a ratio of the demethylated DNA, is also proposed (see Patent document 4).

Moreover, as for metastatic colorectal cancer and primary or metastatic stomach or esophageal cancer, a method of screening expression of SI, CDX1 or CDX2 as an index is proposed (see Patent document 5).

However, a tumor marker is used for understanding dynamics of advanced malignant tumor at present, but a method of inspecting and diagnosing the cancer by using the tumor marker has not been established for an early diagnosis yet.

Especially, it is difficult for the pancreatic cancer to be diagnosed at an early stage, and the treatment results thereof are also too bad. Therefore, it is imperative to develop a method to be used for screening the pancreatic cancer.

## PRIOR ART DOCUMENT

### PATENT DOCUMENT

Patent document 1: JP-A 2000-502902
Patent document 2: JP-A 2003-041843
Patent document 3: JP-A 2001-33460
Patent document 4: JP-A 2002-112799
Patent document 5: JP-A 2003-532389

### NON-PATENT DOCUMENT

Non-patent document 1: Somatic Cell Genet., 5, 957-972, 1979
Non-patent document 2: Cancer Res., 42, 601, 1982
Non-patent document 3: Int. Arch. Allergy Appl. Immunol., 71, 178-181, 1983
Non-patent document 4: The Japanese Journal of Surgery (Nihon Gekagakkai-shi), 87, 236, 1986

## SUMMARY OF THE INVENTION

### PROBLEM TO BE SOLVED BY THE INVENTION

An object of the present invention is to provide a diagnostic marker for digestive organ cancer capable of being used for an inspection method of easily determining whether or not to have a digestive organ cancer at an early stage, and an inspection method for digestive organ cancer of easily determining whether or not to have a digestive organ cancer at an early stage.

### MEANS FOR SOLVING PROBLEM

In order to achieve such an object, the present invention includes the following features (1) to (12).

(1) A diagnostic marker for digestive organ cancer which is used for determining whether or not to have a digestive organ cancer, comprising:

at least one of N-binding type sugar chains released from a glycoprotein contained in blood and represented by the following formulas (1) to (6):

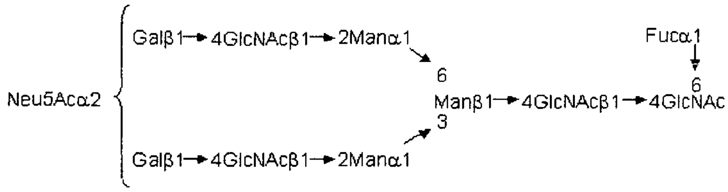

(1)

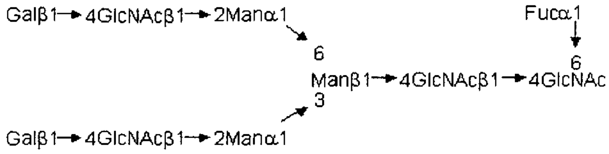

(2)

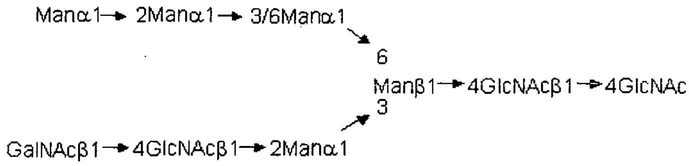

(3)

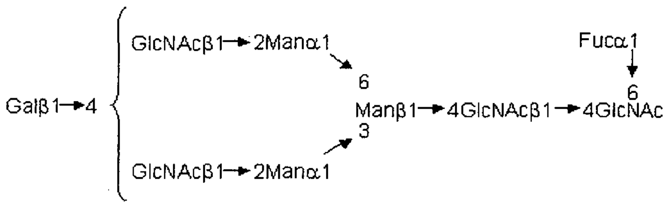

(4)

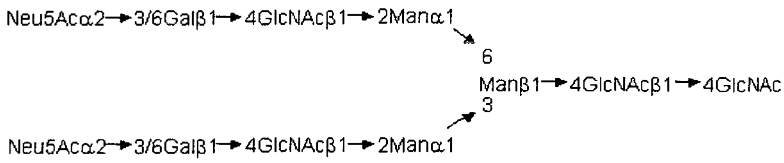

(5)

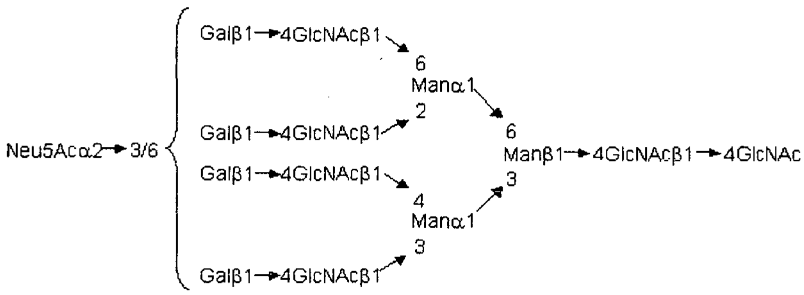

(6)

(2) The diagnostic marker for digestive organ cancer according to the above feature (1), wherein a mass spectrum of the N-binding type sugar chain represented by the formula (1), which is obtained by a mass spectrometry using a MALDI-TOF-MS type analyzer, includes a peak having a mass-to-charge ratio of 2521 m/z.

(3) The diagnostic marker for digestive organ cancer according to the above feature (1), wherein a mass spectrum of the N-binding type sugar chain represented by the formula (2), which is obtained by a mass spectrometry using a MALDI-TOF-MS type analyzer, includes a peak having a mass-to-charge ratio of 2216 m/z.

(4) The diagnostic marker for digestive organ cancer according to the above feature (1), wherein a mass spectrum of the N-binding type sugar chain represented by the formula (3), which is obtained by a mass spectrometry using a MALDI-TOF-MS type analyzer, includes a peak having a mass-to-charge ratio of 2216 m/z.

(5) The diagnostic marker for digestive organ cancer according to the above feature (1), wherein a mass spectrum of the N-binding type sugar chain represented by the formula (4), which is obtained by a mass spectrometry using a MALDI-TOF-MS type analyzer, includes a peak having a mass-to-charge ratio of 2054 m/z.

(6) The diagnostic marker for digestive organ cancer according to the above feature (1), wherein a mass spectrum of the N-binding type sugar chain represented by the formula (5), which is obtained by a mass spectrometry using a MALDI-TOF-MS type analyzer, includes a peak having a mass-to-charge ratio of 2681 m/z.

(7) The diagnostic marker for digestive organ cancer according to the above feature (1), wherein a mass spectrum of the N-binding type sugar chain represented by the formula (6), which is obtained by a mass spectrometry using a MALDI-TOF-MS type analyzer, includes a peak having a mass-to-charge ratio of 3108 m/z.

(8) The diagnostic marker for digestive organ cancer according to the above feature (1), wherein the digestive organ cancer is pancreatic cancer, esophageal cancer or stomach cancer.

(9) An inspection method for digestive organ cancer of determining whether or not to have a digestive organ cancer using the diagnostic marker for digestive organ cancer according to the above feature (1), comprising:

detecting N-binding type sugar chains released from a glycoprotein and represented by the formulas (1) to (6); and
determining whether or not to have the digestive organ cancer based on a detected result in the detecting step.

(10) The inspection method for digestive organ cancer according to the above feature (9), wherein in the detecting step, the N-binding type sugar chains represented by the formulas (1) to (6) are detected by identifying peaks included in a mass spectrum thereof, which is obtained by a mass spectrometry using a MALDI-TOF-MS type analyzer.

(11) The inspection method for digestive organ cancer according to the above feature (10), wherein in the case where a detected value of at least one of a peak having a mass-to-charge ratio of 2521 m/z, a peak having a mass-to-charge ratio of 2216 m/z and a peak having a mass-to-charge ratio of 2054 m/z is lower than a detected value of a normal subject, determined is suspicion of having the digestive organ cancer.

(12) The inspection method for digestive organ cancer according to the above feature (10), wherein in the case where a detected value of at least one of a peak having a mass-to-charge ratio of 2681 m/z and a peak having a mass-to-charge ratio of 3108 m/z is higher than a detected value of a normal subject, determined is suspicion of having the digestive organ cancer.

## EFFECT OF THE INVENTION

By detecting a diagnostic marker for digestive organ cancer according to the present invention, that is, specific N-binding type sugar chains in blood collected from a subject, it becomes possible to determine whether or not to have a digestive organ cancer at an early stage.

Therefore, by an inspection method for digestive organ cancer of determining whether or not to have a digestive organ cancer using such a diagnostic marker for digestive organ cancer, it is possible to easily determine whether or not to have a digestive organ cancer at an early stage, to thereby start to cure the digestive organ cancer from the early stage.

## BRIEF DESCRIPTION OF THE DRAWINGS

FIG. 1 is a graph showing a result of measuring an amount of each sugar chain, which is determined to be a significant difference between a normal subject group and a pancreatic cancer patient group by T-test, contained in each sample (see description of Example with regard to revision of amount of sugar chain).

FIG. 2 is a graph showing a result of measuring an amount of each sugar chain, which is determined to be a significant difference between a normal subject group and an esophageal cancer patient group by T-test, contained in each sample (see description of Example with regard to revision of amount of sugar chain).

FIG. 3 is a graph showing a result of measuring an amount of each sugar chain, which is determined to be a significant difference between a normal subject group and a stomach cancer patient group by T-test, contained in each sample (see description of Example with regard to revision of amount of sugar chain).

FIG. 4 is a view showing a result of identifying a combination of peaks which can separate two groups (pancreatic patient group and normal subject group) from each other by subjecting a sample of each of a pancreatic cancer patient and a normal subject to a mass spectrography to obtain a mass spectrum, and then conducting multivariate analysis of an area of each peak included in the obtained mass spectrum.

## MODE FOR CARRYING OUT THE INVENTION

Hereinafter, description will be made on a diagnostic marker for digestive organ cancer and an inspection method for digestive organ cancer in detail based on preferred embodiments.

<Diagnostic marker for digestive organ cancer>

A sugar chain is a general term of molecules in each of which a plurality of simple sugars such as glucose, galactose, mannose, fucose, xylose, N-acetyl glucosamine, N-acetyl galactosamine and sialic acid and derivatives thereof are linearly bound to each other through a glycoside bond.

The sugar chain is very rich in diversity and is a substance becoming involved in various functions which naturally-occurring organisms have. The sugar chain often exists in vivo as a glycoconjugate by being bound to a protein, a fat and the like, and is one of important components in vivo. It is becoming clear that the sugar chain in vivo deeply involves cellular signal transduction, adjustment of function or interaction of a protein and the like.

Examples of a biological polymer having a sugar chain include a proteoglycan existing in a cell wall of a plant-cell and contributing for stabilization of the cell, a glycolipid influencing cell differentiation, cell proliferation, cell adhesion, cell migration and the like, a glycoprotein influencing cell-to-cell interaction or cell recognition, and the like. A mechanism of controlling advanced and precise vital-reaction in which the sugar chains of these polymers mutually substitute, assist, amplify, adjust and obstruct functions thereof is being gradually clarified.

Moreover, if relation of such a sugar chain to the cell differentiation proliferation, the cell adhesion, immunity and cell canceration is made clear, it is expected that a new spread can be provided by closely relating this sugar chain engineering to medicine, cell engineering or internal organ engineering.

It is becoming clear that, especially, a sugar chain existing in a cell-surface has an important role as a scaffold of various vital-reactions. For example, the sugar chain is regarded as relating to occurrence of diseases due to abnormal interaction with a receptor, infection of AIDS virus, Influenza virus and the like, and invasion of toxins of 0157 strain of E. coli bacteria and cholera toxins into a cell.

Moreover, in some kind of a cancer cell, a peculiar sugar chain expresses on a cell-surface thereof. Therefore, the sugar chain existing on the cell-surface is considered as an important molecule which gives the cell a personality.

Among these sugar chains (glycoproteins), a "N-binding type sugar chain" is a sugar chain bound to a nitrogen atom of an amide group contained in a side chain of an asparagine residue of a protein. Such a sugar chain is also referred to as a N-type sugar chain or an asparagine-binding type sugar chain.

The present inventors focused attention on such a N-binding type sugar chain, especially, a N-binding type sugar chain contained in a blood sample of a digestive organ cancer patient, and attempted to develop a diagnostic marker for digestive organ cancer and an inspection method for digestive organ cancer.

Specifically, after obtaining informed consent from subjects including a pancreatic cancer patient of 17 cases, an esophageal cancer patient of 34 cases and a stomach cancer patient of 22 cases, blood was collected from each subject, and then N-binding type sugar chains contained in plasma were subjected to a mass spectrometry. As a result, the present inventors succeeded in identifying N-binding type sugar chains (hereinafter, merely referred to as "sugar chains" on occasion) whose amounts significantly changed in the blood sample of the subject (cancer patient) as compared with a normal subject, and thus have completed the present invention.

Namely, the identified N-binding type sugar chains can be used as a diagnostic marker for digestive organ cancer which is used for determining whether or not to have a digestive organ cancer. These N-binding type sugar chains are released from a glycoprotein contained in blood and contains at least one of N-binding type sugar chains represented by the following formulas (1) to (6):

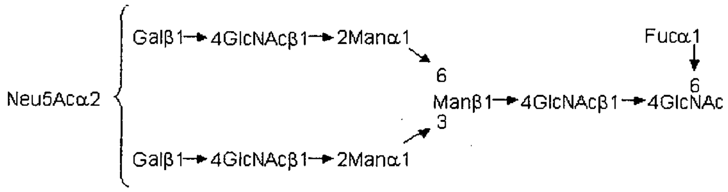

(1)

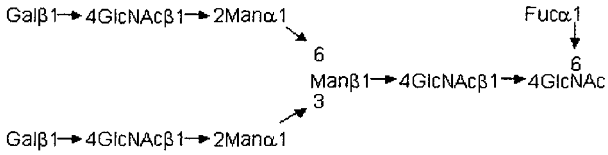

(2)

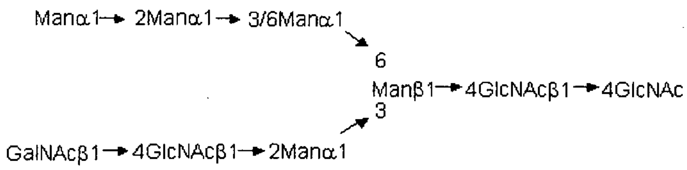

(3)

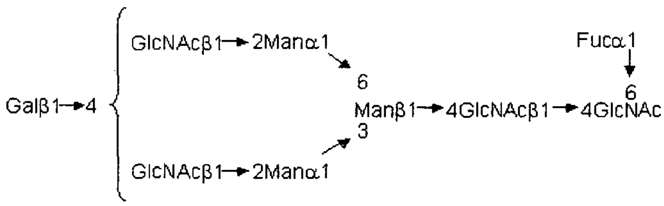

(4)

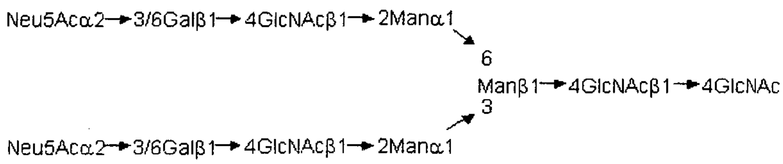

(5)

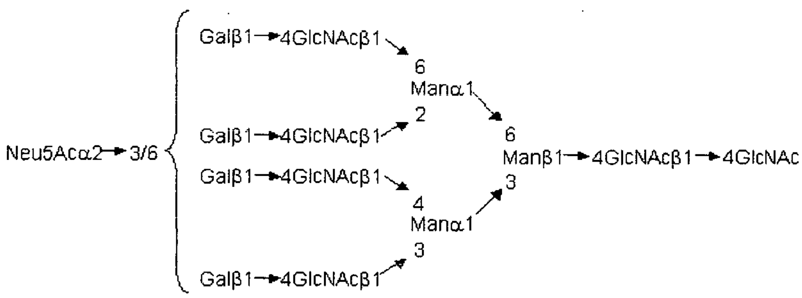

(6)

Such sugar chains can be identified by a mass spectrometry using, for example, a MALDI-TOF-MS type analyzer. A mass spectrum obtained by subjecting each N-binding type sugar chain to the mass spectrometry using the MALDI-TOF-MS type analyzer includes the following characteristic peaks.

Specifically, a mass spectrum of the N-binding type sugar chain represented by the formula (1) includes a peak having a mass-to-charge ratio of 2521 m/z, a mass spectrum of the N-binding type sugar chain represented by the formula (2) includes a peak having a mass-to-charge ratio of 2216 m/z, a mass spectrum of the N-binding type sugar chain represented by the formula (3) includes a peak having a mass-to-charge ratio of 2216 m/z, and a mass spectrum of the N-binding type sugar chain represented by the formula (4) includes a peak having a mass-to-charge ratio of 2054 m/z.

Further, a mass spectrum of the N-binding type sugar chain represented by the formula (5) includes a peak having a mass-to-charge ratio of 2681 m/z, and a mass spectrum of the N-binding type sugar chain represented by the

formula (6) includes a peak having a mass-to-charge ratio of 3108 m/z.

In this regard, in the case where the sugar chains prepared from the blood sample of the subject is subjected to the mass spectrometry using the MALDI-TOF-MS type analyzer, the obtained mass spectrum thereof further includes a peak having a mass-to-charge ratio of 2695 m/z. Therefore, if a N-binding type sugar chain giving origin to such a peak can be identified, this N-binding type sugar chain can be also used as a diagnostic marker for digestive organ cancer which is used for determining whether or not to have a digestive organ cancer.

Further, "MALDI-TOF-MS" is a method of measuring a mass based on time of flight utilizing a MALDI method. Specifically, the MALDI method is a method in which a sample is spotted on a plate, subsequently a matrix solution (2,5-dihydroxybenzoic acid) is added thereto, dried and hardened to be brought into a crystalline state, and then a large amount of energy is applied on the matrix by a pulsed laser irradiation so as to cause desorption of sample-derived ions and matrix-derived ions such as $(M+H)^+$ and $(M+Na)^+$. When the ions are accelerated at a constant accelerating voltage "V", if a mass of the ion is defined as "m", a velocity of the ion is defined as "v", a charge number of the ion is defined as "z", an elementary electric charge is defined as "e", and a time of flight of the ion is defined as "t", a mass-to-charge ratio of the ion (m/z) can be represented by the formula "$m/z = 2eVt^2/L^2$".

Specifically, by using N-binding type sugar chains (diagnostic marker for digestive organ cancer) whose amounts significantly changed in the blood sample of the subject (cancer patient) as compared with the blood sample of the normal subject, it is possible to determine whether or not the subject has a digestive organ cancer based on an inspection method for digestive organ cancer according to the present invention as follows.

<Inspection method for digestive organ cancer>

An inspection method for digestive organ cancer according to the present invention includes: a step of detecting N-binding type sugar chains released from a glycoprotein and represented by the formulas (1) to (6); and a step of determining whether or not to have the digestive organ cancer based on a detected result in the detecting step.

[1] First, N-binding type sugar chains released from a glycoprotein contained in blood and represented by the formulas (1) to (6) are detected (Namely, this step is a detecting step).

[1-1] First, blood is collected from a subject as a sample.

This "blood (sample) collected from subject" may be whole blood containing all blood ingredients, may be also serum or plasma separated from the blood or the like. Among them, as the sample, the serum or plasma is preferable, and the plasma is especially preferable. This makes it possible to detect the N-binding type sugar chains from the sample at excellent detection sensitivity.

[1-2] Next, sugar chains are released from the glycoprotein contained in the blood.

Examples of a releasing method include, but are not limited to, an enzymatic method using N-glycosidase F (also referred to as Glycopeptidase, PN Gase, Glycanase, Glycoamidase or the like), Glycopeptidase A or the like, or a hydrazinolysis method. Among them, as the releasing method, the enzymatic method using the N-glycosidase F can be preferably used. This makes it possible to selectively release the N-binding type sugar chains out of the sugar chains bound to the glycoprotein.

In the case of using such an enzymatic method, a protease such as trypsin can be combined with the above emzyme.

[1-3] Next, the sugar chains released in the blood are purified.

A purifying method is not limited to a specific method, as long as the sugar chains are captured and purified selectively from a mixture in the sample. As this purifying method, preferable is a method in which BlotGlyco (registered trademark) (produced by Sumitomo Bakelite Co., Ltd.) including sugar chain capture beads, which are optimized for highly-sensitive measurements in a MALDI-TOF-MS or high-performance liquid chromatography (HPLC), is used. This makes it possible to purify the sugar chains from the sample at an excellent purifying ratio.

[1-4] Thereafter, the N-binding type sugar chains represented by the above formulas (1) to (6) are detected from the purified sugar chains.

In this regard, such N-binding type sugar chains represented by the above formulas (1) to (6) can be detected by the mass spectrometry using the above mentioned MALDI-TOF-MS type analyzer at high accuracy.

[2] Next, determined is whether or not to have a digestive organ cancer based on a detected result in the detecting step (Namely, this step is a determining step).

Upon subjecting the N-binding type sugar chains in each plasma of the above subjects including the pancreatic cancer patient of 17 cases, the esophageal cancer patient of 34 cases and the stomach cancer patient of 22 cases to the mass spectrometry, a detected value of at least one of peaks having mass-to-charge ratios of 2521, 2216 and 2054 m/z became significantly lower than a detected value of the normal subject.

On the other hand, a detected value of at least one of peaks having mass-to-charge ratios of 2681 and 3108 m/z became significantly higher than a detected value of the normal subject.

Therefore, if a detected value of at least one of the peaks having the mass-to-charge ratios of 2521, 2216 and

2054 m/z is lower than the detected value of the normal subject in a subject, it can be determined that this subject is suspected of having the digestive organ cancer.

Further, if a detected value of at least one of the peaks having the mass-to-charge ratios of 2681 and 3108 m/z is higher than a detected value of the normal subject in a subject, it can be determined that this subject is also suspected of having the digestive organ cancer.

In this regard, a detected value of the peak having the mass-to-charge ratio of 2695 m/z derived from the sugar chain whose structure is not identified became significantly higher than a detected value of the normal subject. Therefore, if this detected value of a subject is higher than the detected value of the normal subject, it can be determined that this subject is also suspected of having the digestive organ cancer.

In this way, by a simple operation of detecting the N-binding type sugar chains represented by the formulas (1) to (6) in the blood, it is possible to determine whether or not to have the digestive organ cancer at an early stage, to thereby start to cure the digestive organ cancer from the early stage.

In this regard, in such an inspection method for digestive organ cancer, by determining whether or not to have the digestive organ cancer using a combination of the detected results of some sugar chains out of the above sugar chains, it is also possible to improve accuracy of the inspection method. Further, by using the inspection method for digestive organ cancer according to the present invention in combination with the other inspection method, it is also possible to improve the accuracy of inspecting the digestive organ cancer.

For example, the following results have been obtained by analysis of the present inventors.

Namely, in two groups of the pancreatic cancer patient group and the normal subject group, N-binding type sugar chains released from a glycoprotein contained in each collected blood were subjected to a mass spectrometry using a MALDI-TOF-MS type analyzer, and then peaks included in an obtained mass spectrum were identified. Thereafter, common peaks included in the mass spectrums obtained in the pancreatic cancer patient group were selected, an area of each of the peaks of the pancreatic cancer patient group and the normal subject group was subjected to multivariate analysis, and then a combination of the peaks capable of separating the above two groups (patient group and normal subject group) from each other was determined. By doing so, it was found that the two groups could be separated from each other, based on detected values of peaks having mass-to-charge ratios of 1326, 1892, 2054, 2172, 2216, 2257, 2334, 2375, 2521, 2639, 2681, 2695, 2703, 2725, 2827, 3030 and 3108 m/z.

Therefore, in the case where in addition to the detected values of the peaks having the mass-to-charge ratios of 2521, 2216, 2054, 2681, 3108 and 2695 m/z, the detected value of at least one of the peaks having the mass-to-charge ratios (1326, 1892, 2172, 2257, 2334, 2375, 2639, 2703, 2725, 2827 and 3030 m/z) different from the above peaks is off from the detected value of the normal subject in a subject, by determining that this subject is strongly suspected of having the digestive organ cancer, it is possible to improve the accuracy of inspecting the digestive organ cancer.

In this regard, a structural formula of the sugar chain giving origin to the peak having the mass-to-charge ratio of 1326 m/z is estimated to be $(Hex)_2(HexNAc)_2(Deoxyhexose)_1$ (see Table 1).

A structural formula of the sugar chain giving origin to the peak having the mass-to-charge ratio of 1892 m/z is estimated to be $(HexNAc)_2(Deoxyhexose)_1 + (Man)_3(GlcNAc)_2$ (see Table 1).

A structural formula of the sugar chain giving origin to the peak having the mass-to-charge ratio of 2172 m/z is estimated to be $(Hex)_2(HexNAc)_1(NeuAc)_1 + (Man)_3(GlcNAc)_2$ (see Table 1).

A structural formula of the sugar chain giving origin to the peak having the mass-to-charge ratio of 2257 m/z is estimated to be $(Hex)_1(HexNAc)_3(Deoxyhexose)_1 + (Man)_3(GlcNAc)_2$ (see Table 1).

A structural formula of the sugar chain giving origin to the peak having the mass-to-charge ratio of 2334 m/z is estimated to be $(Hex)_3(HexNAc)_1(NeuAc)_1 + (Man)_3(GlcNAc)_2$ (see Table 1).

A structural formula of the sugar chain giving origin to the peak having the mass-to-charge ratio of 2375 m/z is estimated to be $(Hex)_2(HexNAc)_2(NeuAc)_1 + (Man)_3(GlcNAc)_2$ (see Table 1).

A structural formula of the sugar chain giving origin to the peak having the mass-to-charge ratio of 2639 m/z is estimated to be $(Hex)_3(HexNAc)_4 + (Man)_3(GlcNAc)_2$ (see Table 1).

A structure of the sugar chain giving origin to the peak having the mass-to-charge ratio of 2703 m/z is estimated to be an adduct of the above sugar chain giving origin to the peak having the mass-to-charge ratio of 2681 m/z (see Table 1).

A structural formula of the sugar chain giving origin to the peak having the mass-to-charge ratio of 2725 m/z is estimated to be $(Hex)_2(HexNAc)_3(Deoxyhexose)_1(NeuAc)_1 + (Man)_3(GlcNAc)_2$ (see Table 1).

A structural formula of the sugar chain giving origin to the peak having the mass-to-charge ratio of 2827 m/z is estimated to be $(Hex)_2(HexNAc)_2(Deoxyhexose)_1(NeuAc)_2 + (Man)_3(GlcNAc)_2$ (see Table 1).

A structural formula of the sugar chain giving origin to the peak that mass-to-charge ratio of 3030 m/z is estimated to be $(Hex)_2(HexNAc)_3(Deoxyhexose)_1(NeuAc)_2 + (Man)_3(GlcNAc)_2$ (see Table 1).

While the diagnostic marker for digestive organ cancer and the inspection method for digestive organ cancer according to the present invention have been described based on the embodiments hereinabove, the present invention

is not limited thereto.

For example, any optional steps may be added to the inspection method for digestive organ cancer according to the present invention as needed.

Further, the "digestive organ cancer" to be determined using the inspection method for digestive organ cancer according to the present invention is not limited to a specific type. However, the present invention is effectively used for determining whether or not to have a refractory cancer such as the pancreatic cancer or the esophageal cancer, and a cancer with high frequency of occurrence such as the stomach cancer.

Furthermore, any analyzers other than the MALDI-TOF-MS type analyzer can be also used as far as they can detect the above sugar chains. For example, as an ion source, a source utilizing an electron ionization method, a chemical ionization method, a field desorption method, a fast atom bombardment method, an electrospray ionization method, an atmospheric pressure chemical ionization method or the like can be used. Further, for example, as an analysis method, a magnetic deflection type method, a quadrupole type method, an ion trap type method, a Fourier transform ion cyclotron resonance type method or the like can be used.

Further, in the present invention, a high-performance liquid chromatography can be also used as far as it can detect the above sugar chains, and the above mass spectrometry and the high-performance liquid chromatography can be also used in combination.

## EXAMPLES

Hereinbelow, the present invention will be described in detail with reference to Examples, but the invention is not restricted thereto.

[Example 1]

(1) Collection of blood

After obtaining informed consent from subjects including a pancreatic cancer patient of 17 cases, an esophageal cancer patient of 34 cases, a stomach cancer patient of 22 cases, and a normal subject of 11 cases as a reference, blood was collected from each subject, and then plasma was separated by centrifugalization. The obtained samples (plasma) were anonymized in a linkable manner, and then kept frozen at -80°C.

(2) Preparation of enzymatically-treated plasma sample

In order to release modified sugar chains from a protein, the plasma of each subject was treated with N-glycosidase F and trypsin. Specifically, pure water (165 μL), a 1M ammonium bicarbonate (25 μL) and a 120 mM dithiothreitol (25 μL) were added to 100 μL of the plasma to obtain a mixture, and the mixture was left at 60°C for 30 minutes. Thereafter, the mixture was added with a 123 mM iodoacetamide (50 μL), and left at room temperature under shade for 1 hour. Subsequently, the mixture was added with trypsin (2000 units, 25 μL), left at 37°C for 1 hour, and then heated at 80°C for 15 minutes to denaturalize the trypsin. After being cooled down to room temperature, the mixture was added with N-glycosidase F (10 units, 10 μL), and left at 37°C overnight. Thereafter the mixture was heated at 80°C for 15 minutes to denaturalize the enzyme. In this way, obtained was an enzymatically-treated plasma sample having a final volume of 400 μL.

Further, an internal standard glucose oligomer (1-20) (Seikagaku Corporation, #800111) was dissolved in pure water so that a concentration thereof was 10 mg/mL, to thereby prepare an internal standard sugar chain solution.

Next, 5 μL (equivalent to 50 μg) of the internal standard sugar chain solution was added to 95 μL of the enzymatically-treated plasma sample to prepare a solution having a total volume of 100 μL. 20 μL of the solution was treated with beads for capturing sugar chains (BlotGlyco (registered trademark) for MALDI (produced by Sumitomo Bakelite Co., Ltd.)) to capture released sugar chains. Thereafter, the captured sugar chains were labeled.

(3) Analysis of sugar chain analytical results

The sugar chains captured by the beads were purified and separated, mixed with a matrix (2,5-dihydroxybenzoic acid) solution, and then subjected to a MALDI-TOF-MS measurement. As a mass analyzer, Autoflex III smartbeam TOF/TOF (produced by Bruker Daltonics Inc.) was used.

Further, a software attached to the above analyzer (e.g., flexControl, flexAnalysis) was used for data collection and analysis. Furthermore, laser ionization was carried out based on a general positive ion mode, and detection was carried out based on a reflector mode.

20 to 100 peaks were identified from the obtained mass spectrum.

For each of the pancreatic cancer, stomach cancer and pancreatic cancer, six sugar chains other than the internal standard sugar chain and the like were selected as an analysis object, and then an amount of each sugar chain was measured based on comparison with the internal standard sugar chain. Further, areas of all peaks (signals) derived from each sugar chain were summed, a ratio among the summed areas of the sugar chains was calculated, and then the measured amount (abundance) of each sugar chain was corrected based on the ratio.

Further, two groups of the normal subject group and the pancreatic cancer patient group, two groups of the normal subject group and the esophageal cancer group and two groups of the normal subject group and the stomach cancer patient group were, respectively, subjected to a T-test, to attempt to identify sugar chains each showing a significant difference.

As a result, with regard to the pancreatic cancer and the esophageal cancer, identified were the sugar chains giving origin to the peaks having the mass-to-charge ratios of 2521, 2216, 2054, 2681, 3108 and 2695 m/z. Further, with regard to the stomach cancer, identified were the sugar chains giving origin to the peaks having the mass-to-charge ratios of 2521, 2216 and 2054 m/z (see FIGs. 1 to 3).

In this regard, with regard to the cancer patient, a ratio of peak area/peak total area (%) of each of the sugar chains giving origin to the peaks having the mass-to-charge ratios of 2521, 2216 and 2054 m/z was significantly low. On the other hand, with regard to the cancer patient, the ratio of each of the sugar chains giving origin to the peaks having the mass-to-charge ratios of 2681, 3108 and 2695 m/z was significantly high.

A correct answer ratio to be obtained by using the sugar chains giving origin to the peaks having the mass-to-charge ratios of 2521 and 2216 m/z was calculated with six classifiers (Compound Covariate Predictor, Diagonal Linear Discriminant Analysis, 1-Nearest Neighbor Predictor, 3-Nearest Neighbor Predictor, Nearest Centroid Predictor, Support Vector Machine Predictor). As a result, a correct answer ratio of about average 88% was obtained with regard to the pancreatic cancer.

Further, estimated was a structural formula of each of the sugar chains from the peaks (signals) included in the mass spectrum obtained by the mass spectrometry.

As a result, the sugar chain giving origin to the peak having the mass-to-charge ratio of 2521 m/z was estimated to have the following structure (1).

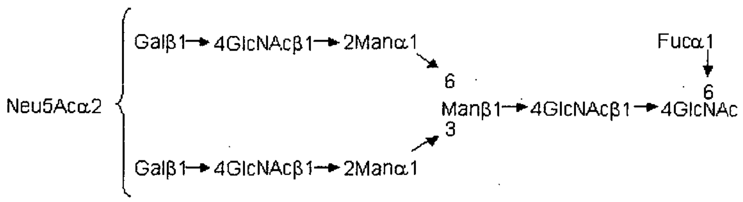
(1)

The sugar chain giving origin to the peak having the mass-to-charge ratio of 2216 m/z was estimated to have the following structure (2) or (3).

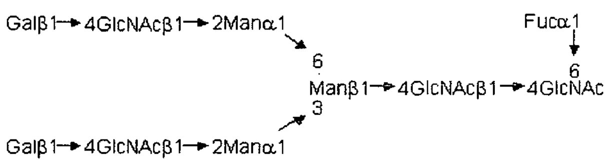
(2)

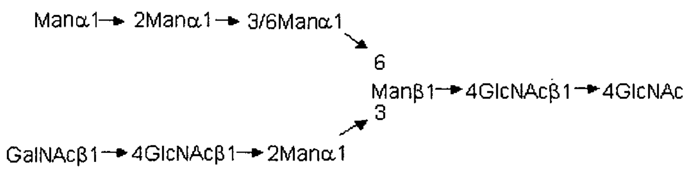
(3)

The sugar chain giving origin to the peak having the mass-to-charge ratio of 2054 m/z was estimated to have the following structure (4).

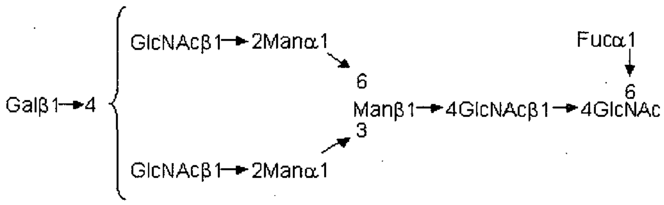

(4)

The sugar chain giving origin to the peak having the mass-to-charge ratio of 2681 m/z was estimated to have the following structure (5).

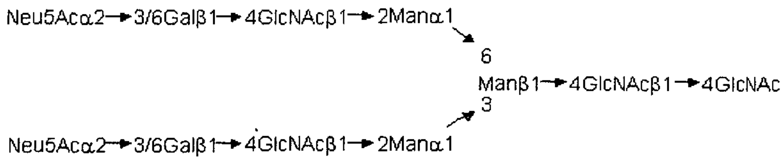

(5)

The sugar chain giving origin to the peak having the mass-to-charge ratio of 3108 m/z was estimated to have the following structure (6).

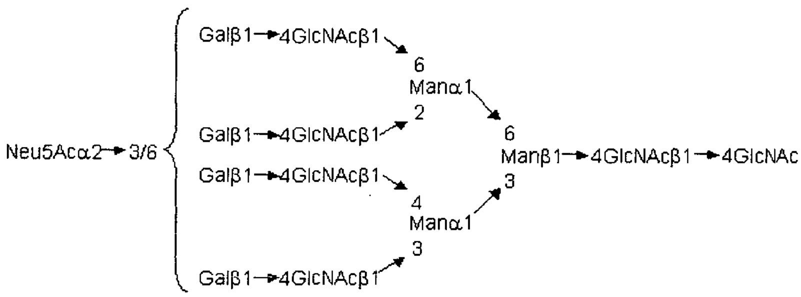

(6)

In this regard, the structure of the sugar chain giving origin to the peak having the mass-to-charge ratio of 2695 m/z could not be estimated.

[Example 2]

(1) Collection of blood

After obtaining informed consent from subjects including a pancreatic cancer patient of 17 cases, and a normal subject of 11 cases as a reference, blood was collected from each subject, and then plasma was separated by centrifugalization. The obtained samples (plasma) were anonymized in a linkable manner, and then kept frozen at - 80°C.

(2) Preparation of enzymatically-treated plasma sample

In order to release modified sugar chains from a protein, the plasma of each subject was treated with N-glycosidase F and trypsin. Specifically, pure water (165 μL), a 1M ammonium bicarbonate (25 μL) and a 120 mM dithiothreitol (25 μL) were added to 100 μL of the plasma to obtain a mixture, and the mixture was left at 60°C for 30 minutes. Thereafter, the mixture was added with a 123 mM iodoacetamide (50 μL), and left at room temperature under shade for 1 hour. Subsequently, the mixture was added with trypsin (2000 units, 25 μL), left at 37°C for 1 hour, and then heated at 80°C for 15 minutes to denaturalize the trypsin. After being cooled down to room temperature, the mixture was added with N-glycosidase F (10 units, 10 μL), and left at 37°C overnight. Thereafter the mixture was heated at 80°C for 15 minutes to denaturalize the enzyme. In this way, obtained was an enzymatically-treated plasma sample having a final volume of 400 μL.

Further, an internal standard glucose oligomer (1-20) (Seikagaku Corporation, #800111) was dissolved in pure water so that a concentration thereof was 10 mg/mL, to thereby prepare an internal standard sugar chain solution.

Next, 5 μL (equivalent to 50 μg) of the internal standard sugar chain solution was added to 95 μL of the enzymatically-treated plasma sample to prepare a solution having a total volume of 100 μL. 20 μL of the solution was

treated with beads for capturing sugar chains (BlotGlyco (registered trademark) for MALDI (produced by Sumitomo Bakelite Co., Ltd.)) to capture released sugar chains. Thereafter, the captured sugar chains were labeled.

(3) Analysis of sugar chain analytical results

The sugar chains captured by the beads were purified and separated, mixed with a matrix (2,5-dihydroxybenzoic acid) solution, and then subjected to a MALDI-TOF-MS measurement. As a mass analyzer, Autoflex III smartbeam TOF/TOF (produced by Bruker Daltonics Inc.) was used.

Further, a software attached to the above analyzer (e.g., flexControl, flexAnalysis) was used for data collection and analysis. Furthermore, laser ionization was carried out based on a general positive ion mode, and detection was carried out based on a reflector mode.

20 to 100 peaks were identified from the obtained mass spectrum. Common peaks between the pancreatic cancer patients were selected. By analyzing an area of each of the peaks of the pancreatic cancer patient group and the normal subject group using a multivariate analysis soft SIMCA-P/P+ (produced by UMETRICS Inc.), it was determined that the two groups (of the pancreatic cancer patient group and the normal subject group) could be separated from each other.

As a result, as the peaks capable of separating the two groups from each other, determined were the peaks having the mass-to-charge ratios of 1326, 1892, 2054, 2172, 2216, 2257, 2334, 2375, 2521, 2639, 2681, 2703, 2725, 2827, 3030 and 3108 m/z (see FIG. 4 and Table 1).

Table 1

| m/z | δmass | Estimated structure of sugar chain |
|---|---|---|
| 1326.5 | 1.974 | $(Hex)_2(HexNAc)_2(Deoxyhexose)_1$ |
| 1892.7 | 0.013 | $(HexNAc)_2(Deoxyhexose)_1$+$(Man)_3(GlcNAc)_2$ |
| 2054.8 | 0.013 | $(Hex)_1(HexNAc)_2(Deoxyhexose)_1$+$(Man)_3(GlcNAc)_2$ |
| 2172.8 | 0.008 | $(Hex)_2(HexNAc)_1(NeuAc)_1$+$(Man)_3(GlcNAc)_2$ |
| 2216.8 | 0.012 | $(Hex)_2(HexNAc)_2(Deoxyhexose)_1$+$(Man)_3(GlcNAc)_2$ |
| 2257.9 | 0.013 | $(Hex)_1(HexNAc)_3(Deoxyhexose)_1$+$(Man)_3(GlcNAc)_2$ |
| 2334.9 | 0.009 | $(Hex)_3(HexNAc)_1(NeuAc)_1$+$(Man)_3(GlcNAc)_2$ |
| 2375.9 | 0.009 | $(Hex)_2(HexNAc)_2(NeuAc)_1$+$(Man)_3(GlcNAc)_2$ |
| 2521.9 | 0.008 | $(Hex)_2(HexNAc)_2(Deoxyhexose)_1(NeuAc)_1$+$(Man)_3(GlcNAc)_2$ |
| 2639.0 | 0.013 | $(Hex)_3(HexNAc)_4$+$(Man)_3(GlcNAc)_2$ |
| 2681.0 | 0.005 | $(Hex)_2(HexNAc)_2(NeuAc)_2$+$(Man)_3(GlcNAc)_2$ |
| 2703.3 | | Adduct of 2681 |
| 2725.0 | 0.009 | $(Hex)_2(HexNAc)_3(Deoxyhexose)_1(NeuAc)_1$+$(Man)_3(GlcNAc)_2$ |
| 2827.0 | 0.004 | $(Hex)_2(HexNAc)_2(Deoxyhexose)_1(NeuAc)_2$+$(Man)_3(GlcNAc)_2$ |
| 3030.1 | 0.004 | $(Hex)_2(HexNAc)_3(Deoxyhexose)_1(NeuAc)_2$+$(Man)_3(GlcNAc)_2$ |
| 3108.8 | 2.689 | $(Hex)_4(HexNAc)_4(NeuAc)_1$+$(Man)_3(GlcNAc)_2$ |

In this regard, symbols shown in Table 1 mean as follows.

$$\delta mass = [\text{Measured value m/z}] - [\text{Theoretical value m/z}]$$

+: A structure shown in the right side of "+" is a basic structure and a structure shown in the left side thereof is an additional structure.

Hex: hexose (mannose)
HexNAc: N-acetyl hexosamine (N-acetyl glucosamine)
Deoxyhexose: fucose

Man: mannose
GlcNAc: N-acetyl glycosamine
NeuAc: N-acetyl neuraminic acid

In this regard, the value of the mass-to-charge ratio (m/z) shown in FIGs. 1 to 4 and Table 1 should be understood that a measurement error within ±1 can occur.

## INDUSTRIAL APPLICABILITY

In the present invention, specific N-binding type sugar chains released from a glycoprotein contained in blood are used as a diagnostic marker for digestive organ cancer which is used for determining whether or not to have a digestive organ cancer. This makes it possible to determine whether or not to have the digestive organ cancer at an early step. Therefore, the present invention has industrial applicability.

## Claims

1. A diagnostic marker for digestive organ cancer which is used for determining whether or not to have a digestive organ cancer, comprising:

at least one of N-binding type sugar chains released from a glycoprotein contained in blood and represented by the following formulas (1) to (6):

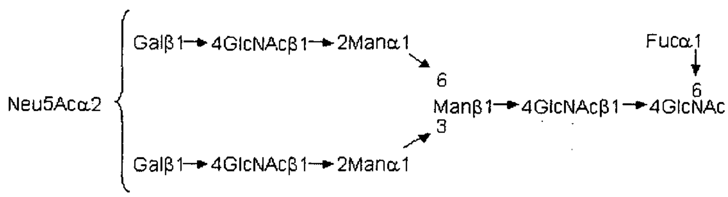
(1)

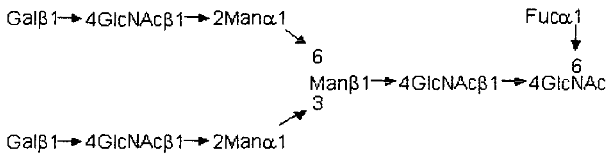
(2)

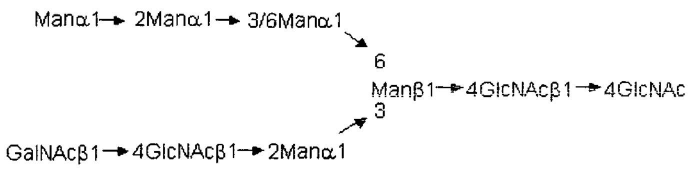
(3)

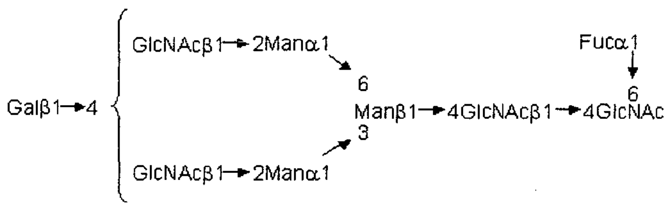
(4)

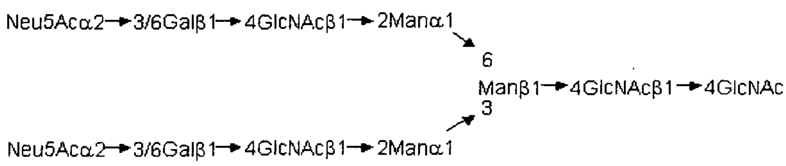
(5)

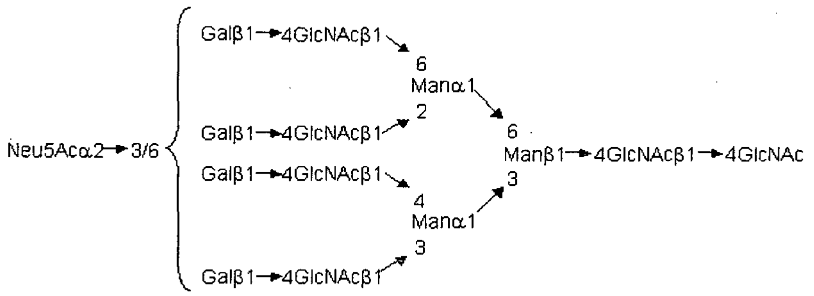 (6)

2. The diagnostic marker for digestive organ cancer as claimed in claim 1, wherein a mass spectrum of the N-binding type sugar chain represented by the formula (1), which is obtained by a mass spectrometry using a MALDI-TOF-MS type analyzer, includes a peak having a mass-to-charge ratio of 2521 m/z.

3. The diagnostic marker for digestive organ cancer as claimed in claim 1, wherein a mass spectrum of the N-binding type sugar chain represented by the formula (2), which is obtained by a mass spectrometry using a MALDI-TOF-MS type analyzer, includes a peak having a mass-to-charge ratio of 2216 m/z.

4. The diagnostic marker for digestive organ cancer as claimed in claim 1, wherein a mass spectrum of the N-binding type sugar chain represented by the formula (3), which is obtained by a mass spectrometry using a MALDI-TOF-MS type analyzer, includes a peak having a mass-to-charge ratio of 2216 m/z.

5. The diagnostic marker for digestive organ cancer as claimed in claim 1, wherein a mass spectrum of the N-binding type sugar chain represented by the formula (4), which is obtained by a mass spectrometry using a MALDI-TOF-MS type analyzer, includes a peak having a mass-to-charge ratio of 2054 m/z.

6. The diagnostic marker for digestive organ cancer as claimed in claim 1, wherein a mass spectrum of the N-binding type sugar chain represented by the formula (5), which is obtained by a mass spectrometry using a MALDI-TOF-MS type analyzer, includes a peak having a mass-to-charge ratio of 2681 m/z.

7. The diagnostic marker for digestive organ cancer as claimed in claim 1, wherein a mass spectrum of the N-binding type sugar chain represented by the formula (6), which is obtained by a mass spectrometry using a MALDI-TOF-MS type analyzer, includes a peak having a mass-to-charge ratio of 3108 m/z.

8. The diagnostic marker for digestive organ cancer as claimed in claim 1, wherein the digestive organ cancer is pancreatic cancer, esophageal cancer or stomach cancer.

9. An inspection method for digestive organ cancer of determining whether or not to have a digestive organ cancer using the diagnostic marker for digestive organ cancer defined by claim 1, comprising:

detecting N-binding type sugar chains released from a glycoprotein and represented by the formulas (1) to (6); and

determining whether or not to have the digestive organ cancer based on a detected result in the detecting step.

10. The inspection method for digestive organ cancer as claimed in claim 9, wherein in the detecting step, the N-binding type sugar chains represented by the formulas (1) to (6) are detected by identifying peaks included in a mass spectrum thereof, which is obtained by a mass spectrometry using a MALDI-TOF-MS type analyzer.

11. The inspection method for digestive organ cancer as claimed in claim 10, wherein in the case where a detected value of at least one of a peak having a mass-to-charge ratio of 2521 m/z, a peak having a mass-to-charge ratio of 2216 m/z and a peak having a mass-to-charge ratio of 2054 m/z is lower than a detected value of a normal subject, determined is suspicion of having the digestive organ cancer.

12. The inspection method for digestive organ cancer as claimed in claim 10, wherein in the case where a detected value of at least one of a peak having a mass-to-charge ratio of 2681 m/z and a peak having a mass-to-charge ratio of 3108 m/z is higher than a detected value of a normal subject, determined is suspicion of having the digestive

organ cancer.

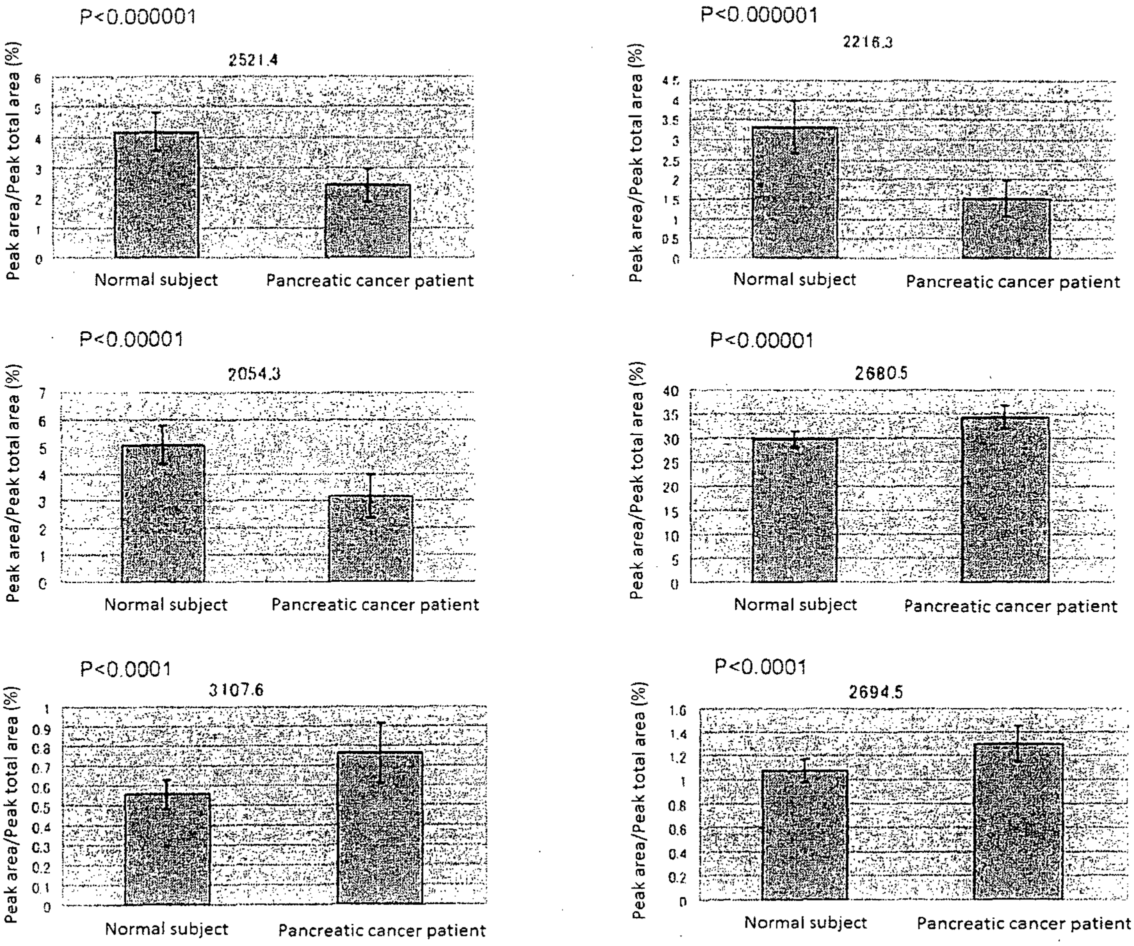
P<0.000001
2521.4
Peak area/Peak total area (%)
Normal subject
Pancreatic cancer patient
P<0.000001
2216.3
Peak area/Peak total area (%)
Normal subject
Pancreatic cancer patient
P<0.00001
2054.3
Peak area/Peak total area (%)
Normal subject
Pancreatic cancer patient
P<0.00001
2680.5
Peak area/Peak total area (%)
Normal subject
Pancreatic cancer patient
P<0.0001
3107.6
Peak area/Peak total area (%)
Normal subject
Pancreatic cancer patient
P<0.0001
2694.5
Peak area/Peak total area (%)
Normal subject
Pancreatic cancer patient

FIG. 1

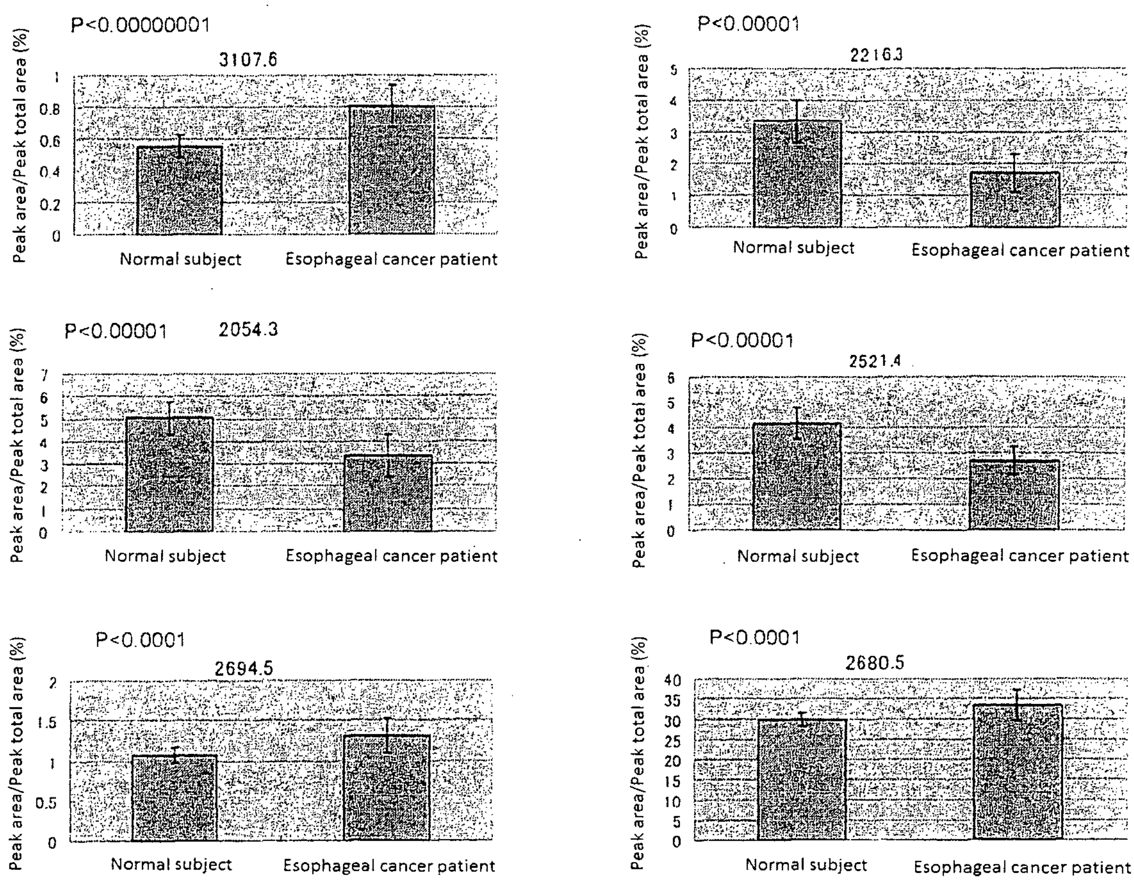
P<0.00000001
3107.6
Peak area/Peak total area (%)
1
0.8
0.6
0.4
0.2
0
Normal subject
Esophageal cancer patient
P<0.00001
2216.3
Peak area/Peak total area (%)
5
4
3
2
1
0
Normal subject
Esophageal cancer patient
P<0.00001
2054.3
Peak area/Peak total area (%)
7
6
5
4
3
2
1
0
Normal subject
Esophageal cancer patient
P<0.00001
2521.4
Peak area/Peak total area (%)
6
5
4
3
2
1
0
Normal subject
Esophageal cancer patient
P<0.0001
2694.5
Peak area/Peak total area (%)
2
1.5
1
0.5
0
Normal subject
Esophageal cancer patient
P<0.0001
2680.5
Peak area/Peak total area (%)
40
35
30
25
20
15
10
5
0
Normal subject
Esophageal cancer patient

FIG. 2

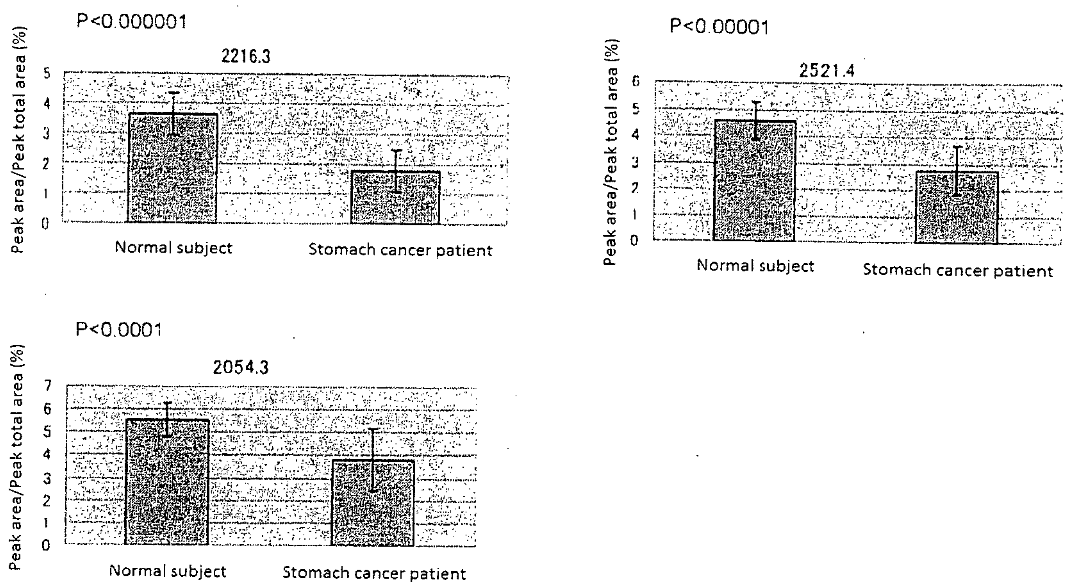

P<0.000001
2216.3
Peak area/Peak total area (%)
Normal subject
Stomach cancer patient
P<0.00001
2521.4
Peak area/Peak total area (%)
Normal subject
Stomach cancer patient
P<0.0001
2054.3
Peak area/Peak total area (%)
Normal subject
Stomach cancer patient


FIG. 3

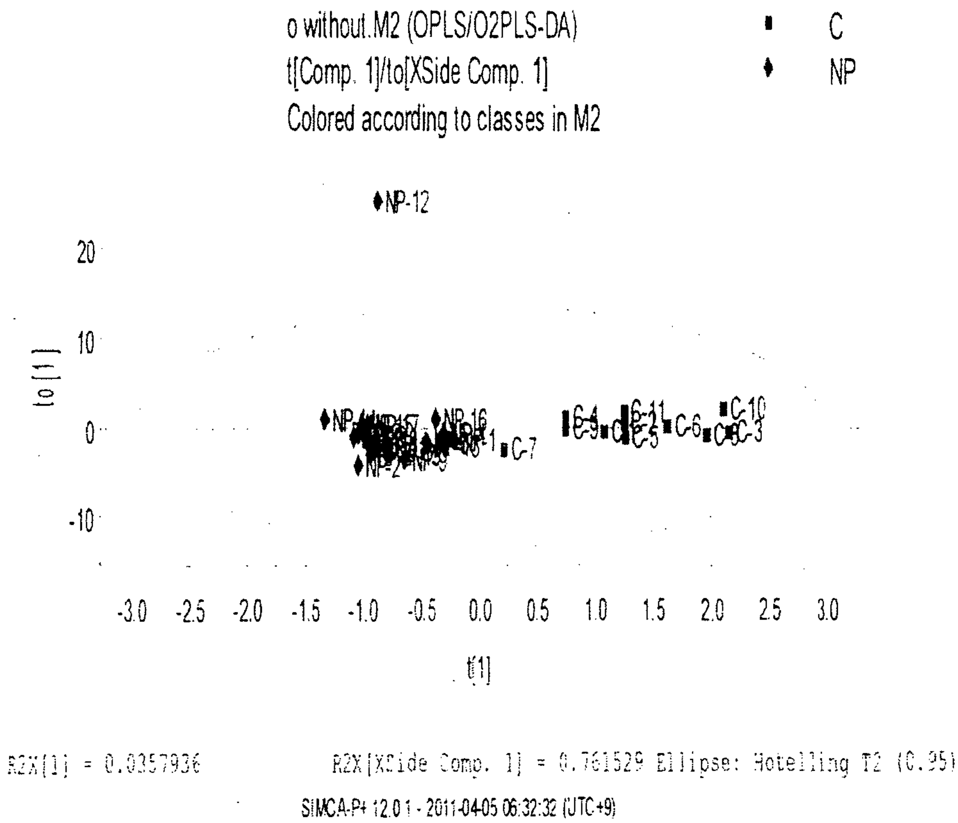
o without.M2 (OPLS/O2PLS-DA)
t[Comp. 1]/to[XSide Comp. 1]
Colored according to classes in M2
C
NP
NP-12
NP-16
C-7
C-4
C-11
C-10
C-6
C-3
NP-2
20
10
0
-10
to[1]
-3.0 -2.5 -2.0 -1.5 -1.0 -0.5 0.0 0.5 1.0 1.5 2.0 2.5 3.0
t[1]
R2X[1] = 0.0357936
R2X[XSide Comp. 1] = 0.761529 Ellipse: Hotelling T2 (0.95)
SIMCA-P+ 12.0.1 - 2011-04-05 06:32:32 (UTC+9)

FIG. 4

REVISED VERSION

## INTERNATIONAL SEARCH REPORT

International application No.
PCT/JP2012/076017

A. CLASSIFICATION OF SUBJECT MATTER
*G01N33/483*(2006.01)i, *G01N27/62*(2006.01)i, *G01N33/66*(2006.01)i

According to International Patent Classification (IPC) or to both national classification and IPC

B. FIELDS SEARCHED

Minimum documentation searched (classification system followed by classification symbols)
G01N33/483, G01N27/62, G01N33/66

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
JSTPlus/JMEDPlus/JST7580(JDreamII), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2009/136506 A1 (Sumitomo Bakelite Co., Ltd.), 12 November 2009 (12.11.2009), claim 1 & JP 2009-270996 A & US 2011/0065141 A1 & EP 2275809 A1 | 1-12 |
| A | Yasuto UCHIKADO et al., "Shokudo Gan no Shinki Shuyo Marker to shite no Kessei Chu N-gata Tosa Profiling", Journal of Japan Surgical Society, 25 May 2011 (25.05.2011), vol.112, special extra issue (1, 2), page 367 | 1-12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 May, 2013 (29.05.13) | 04 June, 2013 (04.06.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No. PCT/JP2012/076017

C (Continuation). DOCUMENTS CONSIDERED TO BE RELEVANT

| Category* | Citation of document, with indication, where appropriate, of the relevant passages | Relevant to claim No. |
|---|---|---|
| A | Fumi ISHIZUKA et al., "Hito Igan Saibo Chu ni Chikuseki suru Yuri N-gata Tosa no Kaiseki", The Japanese Society of Carbohydrate Research Nenkai Yoshishu, 10 July 2007 (10.07.2007), vol.27th, page 94 | 1-12 |
| A | WO 2011/089988 A1 (J-Oil Mills, Inc.), 28 July 2011 (28.07.2011), & JP 4900983 B & EP 2498094 A1 | 1-12 |
| P,X | WO 2011/126053 A1 (Kagoshima University), 13 October 2011 (13.10.2011), claims (Family: none) | 1-12 |
| P,A | Yasuto UCHIKADO et al., "Shokudo Gan Shindan ni Kessei Chu N-gata Tosa o Riyo shita Shinki Shuyo Marker to shite no Kanosei", the Japan Esophageal Society Program · Shorokushu, 2012. 06, vol.66th, page 136 | 1-12 |

Form PCT/ISA/210 (continuation of second sheet) (July 2009)

**INTERNATIONAL SEARCH REPORT**

International application No.
PCT/JP2012/076017

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See next extra sheet.

1. ☒ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (July 2009)

INTERNATIONAL SEARCH REPORT

International application No. PCT/JP2012/076017

Continuation of Box No.III of continuation of first sheet(2)

The technical feature common to the inventions of claims 1-12 is an N-binding sugar chain that serves as a diagnostic marker for digestive system cancer.

The common technical feature, however, cannot be considered as a special technical feature since it does not make a contribution over the prior art in view of the disclosure of document 1 (wherein an N-binding sugar chain is set forth as an esophageal cancer marker).

Further, there is no other same or corresponding special technical feature among these inventions.

Accordingly, the following six inventions (invention groups) are involved in claims.

(Invention 1)

Parts of the inventions of claims 1-12, wherein an N-binding sugar chain represented by formula (1) is used as a digestive system cancer marker.

(Invention 2)

Parts of the inventions of claims 1-12, wherein an N-binding sugar chain represented by formula (2) is used as a digestive system cancer marker.

(Invention 3)

Parts of the inventions of claims 1-12, wherein an N-binding sugar chain represented by formula (3) is used as a digestive system cancer marker.

(Invention 4)

Parts of the inventions of claims 1-12, wherein an N-binding sugar chain represented by formula (4) is used as a digestive system cancer marker.

(Invention 5)

Parts of the inventions of claims 1-12, wherein an N-binding sugar chain represented by formula (5) is used as a digestive system cancer marker.

(Invention 6)

Parts of the inventions of claims 1-12, wherein an N-binding sugar chain represented by formula (6) is used as a digestive system cancer marker.

document 1: Yasuto UCHIKADO et al., "Shokudo Gan no Shinki Shuyo Marker to shite no Kessei Chu N-gata Tosa Profiling", Journal of Japan Surgical Society, 25 May 2011 (25.05.2011), vol.112, special extra issue (1, 2), page 367

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2000502902 A
- JP 2003041843 A
- JP 2001033460 A
- JP 2002112799 A
- JP 2003532389 A

### Non-patent literature cited in the description


- *Somatic Cell Genet.,* 1979, vol. 5, 957-972
- *Cancer Res.,* 1982, vol. 42, 601
- *Int. Arch. Allergy Appl. Immunol.,* 1983, vol. 71, 178-181
- *The Japanese Journal of Surgery (Nihon Geka-gakkai-shi),* 1986, vol. 87, 236